# EUROPÄISCHE PATENTSCHRIFT

(11) **EP 2 682 146 B1**
(45) Veröffentlichungstag und Bekanntmachung des Hinweises auf die Patenterteilung: **17.08.2016**
(21) Anmeldenummer: 12175114.3
(22) Anmeldetag: 05.07.2012
(51) Int. Cl.: A61M 5/46, A61M 5/32, A61M 37/00

(54) **Handgerät zum Tätowieren oder Applizieren von permanentem Make-up und Nadelmodul mit einstellbarer Einstechtiefe**
Handheld device for applying tattoo ink or permanent make-up and needle module with adjustable needle penetration depth
Appareil manuel pour appliquer tatouage ou maquillage permanent et module d'aiguille avec moyen d'ajuster profondeur de pénétration

(43) Veröffentlichungstag der Anmeldung: 08.01.2014
(73) Patentinhaber: MT.DERM GmbH, 14167 Berlin (DE)
(72) Erfinder: Scherkowski, Dirk, Dr.-Ing., 12489 Berlin (DE); Knothe, Kornelius, 12167 Berlin (DE); Loth, Andreas, Dipl.-Ing., 13156 Berlin (DE)
(74) Vertreter: Bittner, Thomas L.

(56) Entgegenhaltungen:
- EP-A1- 2 388 033
- EP-A2- 1 992 387
- EP-A2- 2 454 966
- WO-A1-2012/029082
- US-A- 4 990 135

## Beschreibung

Die Erfindung betrifft ein Nadelmodul zum Stechen einer menschlichen oder tierischen Haut sowie ein Handgeräte mit einem derartigen Nadel modul.

### Hintergrund der Erfindung

Handgeräte werden in verschiedenen Ausgestaltungen bereitgestellt zum Stechen oder Aufstechen einer menschlichen oder tierischen Haut. Es kann vorgesehen sein, dass in Verbindung mit dem Stechvorgang eine Substanz in die Haut oder darunterliegende Gewebe eingebracht wird. Ein solches Einbringen findet beispielsweise beim Nutzen einer Spritze als Handgerät statt, indem durch die Kanüle der Spritze hindurch nach dem Stechen eine zu applizierende Substanz eingebracht wird. Das Einbringen einer Substanz in die Haut findet auch in Verbindung mit dem Herstellen von Tattoos oder permanentem Make-up statt. Hierbei wird die Haut aufgestochen, um dann einen Farbstoff in die Hautschichten einzutragen. Aber auch im Zusammenhang mit anderen medizinischen oder kosmetischen Anwendungen kann vorgesehen sein, in Verbindung mit dem Stechvorgang eine Substanz in die Haut und / oder darunterliegendes Gewebe einzutragen, beispielsweise einen kosmetischen oder einen medizinischen Wirkstoff. Ein Anwendungsfeld in diesem Bereich ist die Mesotherapie.

Unterschiedliche Substanzen erfordern häufig eine unterschiedliche Stechtiefe beim Einstechen der Nadel des Handgerätes in die Haut. Mittels geeigneter Stechtiefe kann beispielsweise die zu entfaltende Wirkung der eingebrachten Substanz optimiert werden. Eine weitere Wirkweise ist die Stimulation der Haut mittels Einstechen in diese, zum Beispiel zur Faltenbehandlung oder zur Narbenbehandlung. Diese Behandlungen können auch ohne Substanzeintrag erfolgen. Eine Einstellbarkeit der Stechtiefe ist hier von Bedeutung, um die gewünschte Hautschicht zu treffen. Ein zu flaches Einstechen liefert nicht die gewünschten Ergebnisse. Ein zu tiefes Einstechen erzeugt zu viel Trauma.

Zur Variation der Einstechtiefe wurde beispielsweise vorgeschlagen, den von der Nadel des Handgerätes ausgeführten Hub zu variieren. Hierdurch ist dann die Länge der Nadel einstellbar, mit welcher diese gegenüber einer vorderen Gehäuseöffnung, zum Beispiel der Öffnung einer Nadeldüse, am Ende der Ausfahrbewegung hervorsteht. Diese Art der Stechtiefeneinstellung erfordert einen Einstellmechanismus zur Hubverstellung und ist deshalb regelmäßig aufwendig.

Das Verstellen eines Nadelüberstandes bei Tattoo- und Permanent Make-up-Geräten kann auch dadurch eingestellt werden, dass Abstand zwischen einer vorderen Nadeldüsenöffnung und der Nadelspitze in der ausgefahrenen Stellung der Nadel verändert wird. Im Dokument EP 2 388 033 A1 wurde ähnlich diesem Prinzip vorgeschlagen, auf einer manuell zu betätigenden Spritze ein Aufsatzteil anzuordnen, dessen axiale Länge einstellbar ist, indem ein vorderes Bauteil über ein Schraubgewinde auf ein Basisbauteil aufgesetzt ist, sodass mittels Drehen des vorderen Bauteils die Länge des Aufsatzteiles insgesamt eingestellt wird. Hierdurch wird schließlich die Länge der Kanüle eingestellt, mit welcher diese über das distale Ende des Aufsatzteiles heraussteht. Auf diese Weise ist die Einstechtiefe für die Kanüle der Spritze einstellbar. Das Einstellen unterschiedlicher Längen des Aufsatzteiles führt dazu, dass das Vorderteil und das Basisteil in unterschiedlichem Umfang mechanisch miteinander gekoppelt sind. Der Überlappungsbereich zwischen dem Vorderteil und dem Basisteil ist umso kürzer, je weiter das Vorderteil herausgeschraubt ist, um die Einstechtiefe zu mindern. Die Verminderung der Überlappung der beiden Bauteile ist nicht vorteilhaft für die mechanische Stabilität der Verbindung zwischen den Bauteilen.

Aus dem Dokument EP 2 454 966 A2 ist eine Tattoo-Vorrichtung bekannt, bei der ein von der Stechnadel ausgeführte Hub einstellbar ist. Hierzu sind an einem Verbindungsstab in axialer Richtung zueinander versetzte Ausnehmungen vorgesehen, in die ein Vorsprung am Nadelschaft eingreift, wobei mittels Eingriff in unterschiedliche Ausnehmungen verschiedene Nadelhübe bewirkt werden. In Abhängigkeit vom Nadelhub ändert sich die Einstechtiefe.

Ein Tattoo-Handgerät ist weiterhin aus dem Dokument EP 1 992 387 A2 bekannt.

Die Dokumente US 4,990,135, WO 2012/029082 A1 sowie EP 2 388 033 A1 beziehen sich auf Spritzen, bei denen eine über die vordere Gehäusespitze vorstehende Nadellänge veränderbar ist.

### Zusammenfassung der Erfindung

Aufgabe der Erfindung ist es, ein Nadelmodul für ein Handgerät zum Stechen einer menschlichen oder einer tierischen Haut anzugeben, bei dem auf einfache Art und Weise eine Stechtiefe flexibel einstellbar ist. Die Einstellbarkeit der Einstechtiefe soll bei unterschiedlichen Arten von Stecheinrichtungen nutzbar sein.

Diese Aufgabe wird erfindungsgemäß durch ein Nadelmodul für ein Handgerät zum Stechen einer menschlichen oder tierischen Haut nach dem unabhängigen Anspruch 1 gelöst. Weiterhin ist ein Handgerät nach dem abhängigen Anspruch 11 geschaffen. Vorteilhafte Ausgestaltungen der Erfindung sind Gegenstand von abhängigen Unteransprüchen.

Die Erfindung umfasst den Gedanken eines Handgerätes zum Stechen einer menschlichen oder tierischen Haut, derart, dass wahlweise auch darunterliegendes Gewebe erreichbar ist. In einem Gehäuse, das vorzugsweise mehrteilig oder -stückig mit trennbaren Gehäuseteilen ausgeführt ist, ist eine Antriebseinrichtung angeordnet, die geeignet ist, wiederholt eine Vorschubbewegung bereitzustellen. Die Antriebseinrichtung kann eine manuelle oder eine motorgetriebene Antriebseinrichtung sein. Charakteristisch ist, dass mithilfe der Antriebseinrichtung wiederholt eine Vorschubkraft bereitgestellt wird, die dann direkt oder über eine zwischengeschaltete Kopplungseinrichtung auf eine Stecheinrichtung gekoppelt wird, um eine von der Stecheinrichtung umfasste Nadel wiederholt auszufahren. Im Fall der motorgetriebenen Antriebseinrichtung kann beispielsweise ein Elektromotor genutzt werden, um mehrfach eine Vorschubkraft bereitzustellen. Dieses kann auf repetierende oder oszillierende Art und Weise erfolgen, wobei in einer Ausgestaltung die Wiederholfrequenz der Vorschubbewegung veränderbar ist. Derartige motorgetriebene Antriebseinrichtungen oder auch andere Antriebseinrichtungen, beispielsweise pneumatische Antriebe, werden beispielsweise in Handgeräten zum Ausbilden eines Tattoos oder von permanentem Make-Up eingesetzt. Aber auch in Verbindung mit dem Einbringen anderer Substanzen, seien es zum Beispiel medizinische oder kosmetische Wirkstoffe in die Haut und / oder darunterliegendes Gewebe, können motorgetriebene oder andere Antriebe für das Handgerät verwendet werden. Derartige Antriebseinrichtungen sind als solche in verschiedenen Ausführungsformen bekannt.

Die von der Antriebseinrichtung bereitgestellte Vorschubbewegung wird auf eine Stecheinrichtung eingekoppelt, die in dem Gehäuse mit einer Nadel gebildet ist, die auch als Stechelement oder Stechmittel bezeichnet werden kann. Das Einkoppeln der Antriebs- oder Vorschubbewegung kann direkt auf die Stecheinrichtung erfolgen oder indirekt über eine zwischengeschaltete Kopplungseinrichtung.

Ergänzend oder alternativ kann ein Rückstellmechanismus vorgesehen sein, um die Nadel nach dem Ausfahren wieder zurückzuholen, beispielsweise ein Feder- und / oder ein Membranmechanismus. Im Betrieb bewegt sich die Nadel und wahlweise zusätzlich an die Nadel koppelnde Bauteile relativ zur Gehäuseöffnung hin und von ihr weg, wobei die Gehäuseöffnung an einer Nadeldüse gebildet ist. Die Relativverlagerung der Nadel kann so geschehen, dass zumindest in einer vollständig ausgefahrenen Stellung der Nadel deren Nadelspitze (Stechelementspitze) an der Öffnung vorn übersteht.

An die Stecheinrichtung koppelt ein die Nadel ganz oder teilweise umgreifender Stechtiefeneinsteller, derart, dass der Stechtiefeneinsteller relativ zur Nadelspitze verlagerbar ist, vorzugsweise in axialer Richtung der Nadel, und beim Vor- und Zurückbewegen der Nadel im Betrieb mit dieser mitbewegt wird. Dieses bedeutet, dass sich die Relativlage von Stechtiefeneinsteller und Nadelspitze beim Vor- und Zurückbewegen der Nadel in Betrieb nicht ändert, nachdem dieser Abstand zuvor eingestellt wurde. Er ist mittels Verlagerung des Stechtiefeneinstellers veränderbar, insbesondere mittels Axialverlagerung, um dann im nachfolgenden Betrieb mit einer geänderten Stechtiefe zu arbeiten. Hierbei ist keine Veränderung des von der Antriebseinrichtung zur Verfügung gestellten Hubes notwendig, auch wenn dieses ergänzend vorgesehen sein kann. Vielmehr wird die Einstechtiefe durch die Relativlage des Stechtiefeneinstellers in Bezug zur Nadelspitze (freie Nadellänge) festgelegt, worauf beide im Betrieb gemeinsam dem von der Antriebseinrichtung bereitgestellten Hub entsprechend bewegt werden.

Der Begriff Nadel in der hier verwendeten Bedeutung steht allgemein für unterschiedliche Arten von Stechelementen oder -werkzeugen, die geeignet sind, eine Haut aufzustechen und bedarfsweise zu durchstechen, wahlweise auch darunterliegende Gewebeabschnitt, wozu insbesondere die folgenden Bauarten gehören: Einzelnadel, Nadelbündel, Einzelkanüle, Kanülenbündel oder -gruppe sowie Nadelplatte. Die Nadeln können hinsichtlich ihrer Längserstreckung gerade oder gekrümmt sein. Bei einer Nadelplatte sind auf einer sich flächig erstreckenden Nadelplatte vorderseitig mehrere Nadeln oder Nadelbündel beabstandet zueinander angeordnet. Diese Nadeln oder Nadelbündel auf der Nadelplatte bewegen sich im Betrieb gemeinsam relativ zur Öffnung der Nadeldüse, nämlich auf diese zu und von ihr weg. Bevorzugt stehen hierbei wenigstens die Nadelspitzen in der ausgefahrenen Stellung vorderseitig von der Fläche der Öffnung der Nadeldüse ab, sodass ein Einstechen in die Haut wenigstens teilweise außerhalb der Nadeldüse stattfindet. Alternativ kann vorgesehen sein, dass Nadelspitze auch in der ausgefahrenen Stellung noch hinter der Öffnung der Nadeldüse zurücksteht, also noch in der Nadeldüse angeordnet ist. Das Aufstechen der Haut findet hierbei statt, wenn die Hautoberflächen sich in die Öffnung der Nadeldüse hinein wölbt bis zur Nadelspitze hin, sei es zum Beispiel aufgrund des Aufsetzens der Nadeldüse auf die Haut und / oder aufgrund einer Sogwirkung, die in der Nadeldüse mittels Anlegen eines Unterdrucks erzeugt wird. Der Stechtiefeneinsteller bewegt sich mit der Nadelplatte beim Aus- und Einfahren ebenfalls mit.

In den Fällen der Verwendung von Kanülen wird ein Wirkstoff durch die Kanüle(n) in die Haut oder darunter liegende Gewebe eingespritzt. Es besteht dann eine Fluidverbindung zu Bauteilen, welche der Druckbeaufschlagung und der Kontrolle des Ausbringens dienen. Solche Fluidanordnungen und Bauteile sind als solche in verschiedenen Ausgestaltungen bekannt.

Die Nadeldüse bildet bei dem Handgerät einen vorderen Abschnitt des Gehäuses, der wahlweise lösbar am Gehäuse angeordnet ist, mit der Öffnung, durch welche hindurch die Nadel in Betrieb wahlweise austritt.

Das Nadel- oder Stechelementmodul für ein Handgerät zum Stechen einer menschlichen oder tierischen Haut umfasst ein Modulgehäuse mit einem Koppelabschnitt zum Koppeln an eine Antriebseinrichtung sowie eine Stecheinrichtung, die direkt oder über eine Kopplungseinrichtung indirekt an die Antriebseinrichtung koppelbar ist, derart, dass eine von der Antriebseinrichtung wiederholt bereitgestellte Vorschubbewegung auf die Stecheinrichtung einleitbar ist. In einer vorderen Öffnung an dem Gehäuse bewegt sich die Nadel und der daren gekoppelle Stechtiefeneinsteller im Betrieb vor und zurück. Die Öffnung ist mittels einer Nadel- oder Stechelementdüse an dem Modulgehäuse gebildet. Die Nadel- oder Stechelementdüse kann lösbar am Modulgehäuse aufgenommen sein.

Ein Stechtiefeneinsteller koppelt an die Nadel- oder Stechelementeinrichtung, derart, dass der Stechtiefeneinsteller in axialer Richtung der Nadel (des Stechelementes) relativ zur Spitze der Nadel verlagerbar ist und beim Vor- / Zurückbewegen der Nadel in Betrieb mit bewegt wird.

Das Nadel- oder Stechelementmodul kann als Einwegmodul ausgeführt sein, um es zum Gebrauch an die Antreibseinrichtung zu koppeln und nach der Benutzung abzunehmen und zu entsorgen. Bevorzugt ist das Einwegmodul steril verpackt.

Es ist vorgesehen, dass sich die Nadel im Betrieb in der Gehäuseöffnung vor und zurück bewegt, wobei die Nadelspitze hierbei durch die Öffnung hindurch treten kann.

Eine bevorzugte Weiterbildung sieht vor, dass die Nadel an einem Nadelträger aufgenommen ist, der direkt oder über die Kopplungseinrichtung indirekt an die Antriebseinrichtung koppelt. Der Nadel- oder Stechelementträger kann beispielsweise einen Nadelschaft (Stechelementschaft) aufweisen, in welchem die Nadel aufgenommen ist. Im Fall der Ausbildung der Stecheinrichtung mit einer Nadelplatte ist diese am Nadelträger aufgenommen, vorzugsweise verkippbar quer zu Axialrichtung der Nadeleinrichtung. Im Fall der direkten Kopplung der Stecheinrichtung an die Antriebseinrichtung kann der Nadelträger eine Art Zwischenstück zwischen Antriebseinrichtung und Stecheinrichtung bilden. In einer Ausgestaltung kann vorgesehen sein, den Nadelträger als Teil der Kopplungseinrichtung für die indirekte Kopplung zwischen Antriebseinrichtung und Stecheinrichtung zu bilden.

Bei einer zweckmäßigen Ausgestaltung kann vorgesehen sein, dass der Stechtiefeneinsteller an der Nadel in axialer Richtung verlagerbar aufgenommen ist. Bei dieser Ausgestaltung kann der Stechtiefeneinsteller auf der Nadel angeordnet sein. Alternativ kann eine Lagerung des Stechtiefeneinstellers am Nadelträger vorgesehen sein. Auch eine gemeinsame Lagerung des Stechtiefeneinstellers auf der Nadel und an dem Nadelträger kann vorgesehen sein. Im Fall der Nutzung einer Nadelplatte kann beispielsweise vorgesehen sein, dass der Stechtiefeneinsteller an die Nadelplatte koppelt, zum Beispiel über ein entlang des Umfangs der Nadelplatte verlaufendes Schraubgewinde, welches zum Verstellen des Stechtiefeneinstellers dient.

Eine vorteilhafte Ausführungsform sieht vor, dass der Stechtiefeneinsteller an der Nadel an einem Nadel- oder Stechelementstößel in axialer Richtung verlagerbar aufgenommen ist. In einer Ausführungsform sitzt der Nadelstößel fest auf der Nadel und bildet die Basis für die Ankopplung des Stechtiefeneinstellers hieran, der seinerseits an dem Nadelstößel lagert.

Bevorzugt sieht eine Fortbildung der Erfindung vor, dass der Stechtiefeneinsteller über einen Drehstellmechanismus an die Stecheinrichtung koppelt. Ein Drehstellmechanismus ist beispielsweise mittels eines Schraubgewindes hergestellt, derart, dass am Drehstellmechanismus beteiligte Bauteile mittels Drehen in ihrer Relativlage zueinander positioniert werden. Beispielsweise kann in einer Ausgestaltung vorgesehen sein, dass der Nadelstößel mit einem Außengewinde versehen ist, auf welches der Stechtiefeneinsteller aufgeschraubt ist, derart, dass mittels Drehen des Stechtiefeneinstellers dessen Relativlage zum Nadelstößel und somit zur Nadel in axialer Richtung der Nadel einstellbar ist. Mittels Drehen wird hierbei der Stechtiefeneinsteller näher in Richtung Nadelspitze verlagert, oder von dieser weg.

Die Gehäuseöffnung ist an einer Nadeldüse gebildet. Bei dem Handgerät ist dann zum Beispiel eine einen vorderen Gehäuseabschnitt bildende Nadeldüse vorgesehen, die eine Öffnung aufweist, in welcher sich die Nadel (das Stechelement) der Stecheinrichtung und hieran koppelnde Bauteile wie zum Beispiel der Stechtiefeneinsteller im Betrieb vor- und zurückbewegt. Bei dieser oder anderen Ausführungen kann vorgesehen sein, dass die Nadel auch beim Zurückbewegen von der Antriebseinrichtung auf dem zurückgelegten Weg wenigstens abschnittsweise zwangsgeführt wird, indem von der Antriebseinrichtung eine Rückzugskraft auf die Nadel eingekoppelt wird.

Bei einer vorteilhaften Ausgestaltung kann vorgesehen sein, dass einem an dem Stechtiefeneinsteller gebildeten Führungselement eine an der Nadeldüse gebildete Führung zugeordnet ist, in welcher das Führungselement beim Vor- / Zurückbewegen der Nadel im Betrieb geführt ist. In einer Ausgestaltung ist vorgesehen, dass an dem Stechtiefeneinsteller ein oder mehrere radial abstehende Führungselemente gebildet sind, die in eine jeweils zugeordnete Führungsnut eingreifen, welche in der Nadeldüse gebildet ist. Es kann vorgesehen sein, dass ein distales Ende der Führung in der Nadeldüse eine Ausfahrbegrenzung für den Stechtiefeneinsteller und somit für die Ausfahrbewegung der Nadel bildet.

Eine Weiterbildung kann vorsehen, dass ein Nadeldüsengehäusebauteil der Nadeldüse verlagerbar an dem Gehäuse angeordnet ist und funktionell an den Stechtiefeneinsteller koppelt, derart, dass mittels Verlagern des Nadeldüsengehäusebauteils der Stechtiefeneinsteller in axialer Richtung relativ zur Nadelspitze verlagert wird. Beispielsweise kann ein Verschieben oder ein Verdrehen des Nadeldüsenbauteils oder der gesamten Nadeldüse aufgrund der funktionellen Kopplung des Stechtiefeneinstellers hieran zu Axialverlagerung des Stechtiefeneinstellers relativ zur Nadelspitze genutzt werden.

Eine bevorzugte Weiterbildung sieht vor, dass das Nadeldüsenbauteil drehbar an dem Gehäuse angeordnet ist. Bei dieser Ausgestaltung kann vorgesehen sein, dass beim Drehen des Nadeldüsengehäusebauteils der Stechtiefenversteller mitgenommen wird und hierdurch aufgrund einer Gewindeverbindung des Stechtiefeneinstellers mit der Nadel oder dem Nadelträger die axiale Relativlage des Stechtiefeneinstellers in Bezug zur Nadelspitze verändert wird, um auf diese Weise eine Stechtiefeneinstellung vorzunehmen.

Zumindest ein distales Ende des Stechtiefeneinstellers umgreift die Nadel wenigstens abschnittsweise. Das Umgreifen der Nadel oder des Stechelementes im Bereich des distalen Endes des Stechtiefeneinstellers kann durchgehend oder unterbrochen sein. Zum Beispiel ist der Stechtiefeneinsteller in einer Ausgestaltung als eine Art Hülse ausgeführt, durch die hindurch die Nadel geführt ist. Bei dieser Ausgestaltung umgreift der Stechtiefeneinsteller die Nadel vorzugsweise entlang seiner gesamten Länge. Form, Größe und Abmessungen des Stechtiefeneinstellers sind an die jeweils umfasste Nadel sowie die gewünschte Genauigkeit bei der Stechtiefenbegrenzung anpassbar. Das distale Ende des Stechtiefeneinstellers kann eine umgekehrte Trichterform aufweisen. Die Formgebung kann hierbei einer beim Einstechen in die Haut auftretenden Gewebedellen nachgebildet sind.

Eine Ausführung sieht vor, dass das distale Ende des Stechtiefeneinstellers einen sich verjüngenden Endabschnitt aufweist. Die Verjüngung ist in Richtung der Öffnung hin gebildet, die der Nadelspitze zugeordnet ist. Beispielsweise ist mittels der Verjüngung ein konischer oder konusähnlicher Endabschnitt gebildet.

Eine distale Endfläche des Stechtiefeneinstellers bildet in einer Betriebsstellung eine gemeinsame Anschlagfläche mit einer vorderseitigen Endfläche der Nadeldüse. Hierbei kann vorgesehen sein, dass die gemeinsame Anschlagfläche eine konische Ausbildung aufweist, wozu die Endfläche des Stechtiefeneinstellers einerseits und die vorderseitige Endfläche der Nadeldüse andererseits beitragen.

Bevorzugt sieht eine Fortbildung vor, dass die Nadeleinrichtung, die Nadeldüse und der Stechtiefeneinsteller an einem Nadel- oder Stechelementmodul gebildet sind, welches lösbar an dem Gehäuse angeordnet ist. Das Nadelmodul kann als Einwegmodul ausgeführt sein. Vorzugsweise ist es rückseitig, also auf der der Antriebseinrichtung zugewandten Seite abgedichtet, derart, dass eine mit der Nadel in der Nadeldüse in Kontakt kommende Flüssigkeit auf der Rückseite nicht aus dem Nadelmodul austreten und in die Antriebseinrichtung gelangen kann. Wahlweise können Teile der Kopplungseinrichtung in das Nadelmodul integriert sein. Es kann vorgesehen sein, zur rückseitigen Abdichtung des Nadelmoduls eine Membran aus einem elastischen Material zu verwenden, die vorzugsweise eine U-Form aufweist. An der Membran kann der Nadelträger abgedichtet aufgenommen sein. Mithilfe der Membran aus elastischem Material kann in einer Ausgestaltung eine Rückstellkraft für die Nadel zum Zurückholen nach der Ausfahrbewegung bereitgestellt werden. Die elastische Membran wird beim Ausfahren der Nadel gestreckt und zieht dann die Nadel wieder zurück. Diese Rückstellkraft kann für sich allein oder in Kombination mit anderen Rückholmechanismen das Wiedereinfahren der Nadel bewirken.

Bei einer vorteilhaften Ausgestaltung kann vorgesehen sein, dass der Stechtiefeneinsteller in einer oder mehreren axialen Verlagerungsstellungen relativ zur Nadelspitze der Nadel fixierbar ist. Beispielsweise können verschiedene Raststellungen des Stechtiefeneinstellers bei der axialen Verlagerung vorgesehen sein.

### Beschreibung bevorzugter Ausführungsformen

Im Folgenden werden bevorzugte Ausführungsbeispielen unter Bezugnahme auf Figuren einer Zeichnung näher erläutert. Hierbei zeigen
- Fig. 1a bis 1d: eine schematische Darstellung eines Handgerätes zum Stechen in eine menschliche oder tierische Haut,
- Fig. 2: eine schematische Darstellung eines vorderen Abschnittes des Handgerätes mit einer ersten Einstechtiefeneinstellung,
- Fig. 3: eine schematische Darstellung eines vorderen Abschnittes des Handgerätes mit einer zweiten Einstechtiefeneinstellung und
- Fig. 4: eine schematische Darstellung eines vorderen Abschnitts eines Handgerätes zum Aufstechen einer Haut.

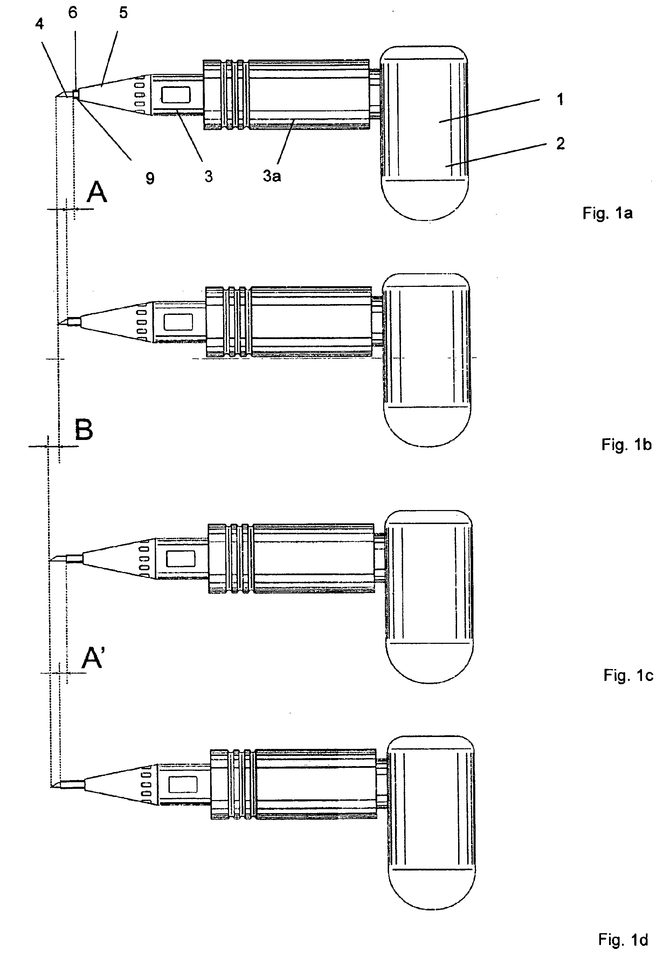

Fig. 1a bis 1d zeigen jeweils eine schematische Darstellung eines Handgerätes zum Stechen einer menschlichen oder tierischen Haut, welches in der dargestellten Ausführungsform modulartig aufgebaut ist, mit einem Gehäuse 1, welches in der gezeigten Ausgestaltung mehrstückig ist und eine Antriebseinrichtung 2 und ein Nadelmodul 3 aufweist. Die Antriebseinrichtung 2 ist geeignet, wiederholt eine Vorschubbewegung bereitzustellen, beispielsweise mittels eines Elektromotors oder manuell, die dann direkt oder indirekt über eine Kopplungseinrichtung (nicht dargestellt) in eine Stecheinrichtung in dem Nadelmodul 3 eingekoppelt wird, um eine Nadel oder ein Stechelement der Stecheinrichtung wiederholt auszufahren.

Ein Griffstück 3a ist lösbar oder nicht lösbar mit dem Nadelmodul 3 und / oder der Antriebseinrichtung 2 verbunden, wobei sich Teile der Antriebseinrichtung 2 und / oder eines Kopplungsmechanismus zum Übertragen der Antriebsbewegung in dem Griffstück 3a erstrecken können.

Eine Nadel 4, die Teil einer Stech- oder Nadeleinrichtung ist, erstreckt sich in einem eine Nadeldüse 5 bildenden vorderen Gehäuseabschnitt und tritt wenigstens beim Stechen der Haut durch eine Öffnung 6 aus. Die Fig. 1a bis 1d zeigen die Nadel 4 in einer teilweise oder ganz ausgefahrenen Stellung, in welcher das Stechen in die Haut stattfindet. Bei Ausfahren wird ein Stechtiefeneinsteller 9 mit der Nadel mitbewegt. Der Überstand der Nadel 4 über das vordere Ende des Stechtiefeneinstellers 9 bestimmt die Einstechtiefe in die Haut. Es ergibt sich, dass die Einstechtiefe bei den Ausführungen in den Fig. 1a und 1c größer als bei den Ausgestaltungen in den Fig. 1b und 1d ist.

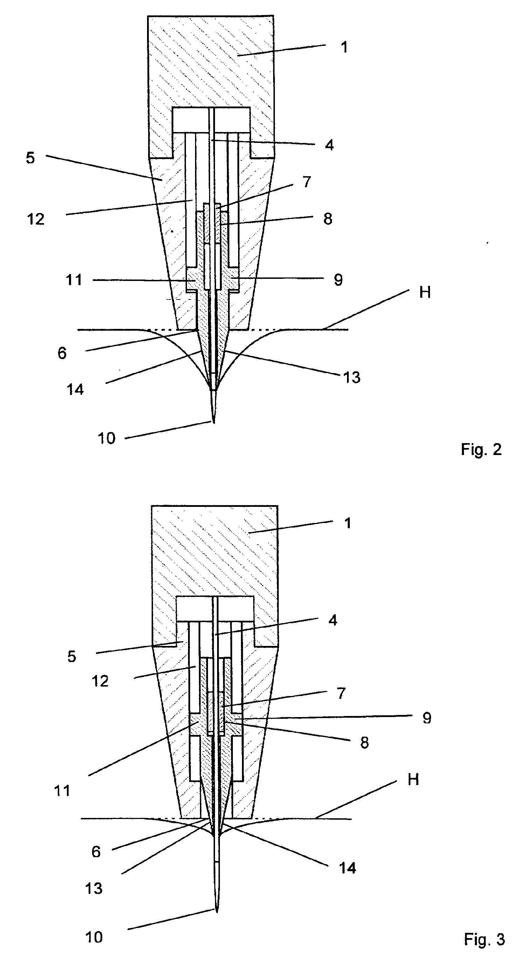

Die Fig. 2 und 3 zeigen einen vorderen Gehäuseabschnitt des Handgerätes aus Fig. 1 im Querschnitt. Die Nadel 4 erstreckt sich in der Nadeldüse 5 und tritt bei der gezeigten Ausführung wenigstens beim Stechen der Haut durch die Öffnung 6 aus. Sowohl die Fig. 2 als auch die Fig. 3 zeigen die Nadel 4 in einer ausgefahrenen Stellung, in welcher das Stechen in die Haut H in unterschiedlicher Tiefe stattfindet.

Auf der Nadel 4 ist ein Nadelstößel 7 angeordnet, welcher über ein Schraubgewinde 8 an einen Stechtiefeneinsteller 9 koppelt. Die axiale Relativlage des Stechtiefeneinstellers 9 zur Nadelspitze 10 der Nadel 4 ist einstellbar, indem der Stechtiefeneinsteller 9 gedreht wird. Dieses erfolgt bei der dargestellten Ausführungsform dadurch, dass der vordere Gehäuseabschnitt, mit dem die Nadeldüse 5 gebildet ist, drehbar gelagert ist und am Stechtiefeneinsteller 9 gebildete Führungselemente 11 in einer zugeordneten Führung 12 der Nadeldüse lagern, wodurch beim Drehen der Nadeldüse der Stechtiefeneinsteller 9 mitgenommen wird, worauf sich dessen axiale Lage in Bezug auf die Nadelspitze 10 verändert.

Ein distaler Endabschnitt 13 des Stechtiefeneinstellers 9 weist eine konische Formgebung auf mit einer Außenfläche 14, an die sich die Haut im Stechprozess anlegt.

Die unterschiedliche Einstichtiefe bei den Ausführungen in den Fig. 2 und 3 ist bestimmt durch die Relativlage des Stechtiefeneinstellers 9 zur Nadelspitze 10.

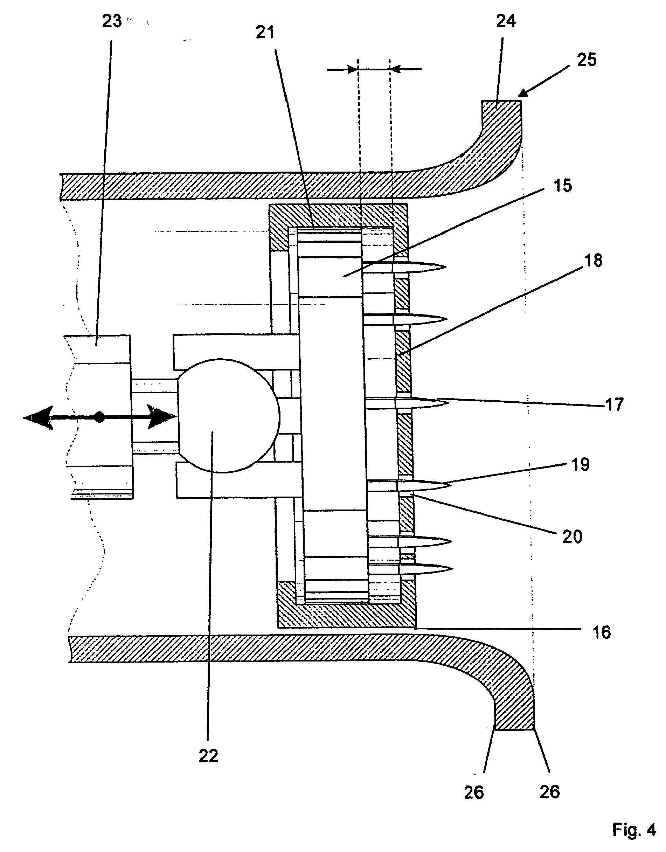

Fig. 4 zeigt eine schematische Darstellung eines vorderen Abschnitts eines Handgerätes zum Aufstechen einer Haut, bei dem im Unterschied zu den vorangehenden Ausführungsformen die Stech- oder Nadeleinrichtung anstelle der Nadel 4 nun eine Nadelplatte 15 aufweist, die im Betrieb in einer Öffnung 16 vor- und zurückbewegt wird. An der Nadelplatte 15 sind mehrere Nadeln 17 vorderseitig angeordnet. An der Nadelplatte 15 ist weiterhin ein Stechtiefeneinsteller 18 aufgenommen, welcher relativ zur Nadelplatte 15 in axialer Richtung verlagerbar ist, beispielweise dadurch, dass der Stechtiefeneinsteller 18 über eine Gewindeverbindung 21 drehbar an der Nadelplatte 15 aufgenommen ist, sodass ein Überstand 19 (freie Nadellänge) der Nadeln 17 über Öffnungen 20 einstellbar ist, um eine Einstechtiefe zu variieren. Im Betrieb werden dann die Nadelplatte 15 sowie der Stechtiefeneinsteller 18 gemeinsam vor- und zurückbewegt.

Bei der dargestellten Ausführungsform ist die Nadelplatte 15 über ein Kugelgelenk 22 an einem Nadelplattenstößel 23 aufgenommen, wodurch ein Verkippen der Nadelplatte 15 relativ zum Nadelstößel 23 ermöglicht ist.

Im Betrieb wird ein vorderer Gehäuseabschnitt 24 mit der Stirnfläche 25 auf die zu stechende Haut aufgelegt. Sodann wird die Nadelplatte 15 zusammen mit dem Stechtiefeneinsteller 18 vor- und zurückgefahren, derart, dass die Nadelspitzen in die Haut eindringen.

An dem vorderen Gehäuseabschnitt 24 können Ecken 26 und / oder Kanten abgerundet sein.

## Patentansprüche

1. Nadelmodul (3) für ein Handgerät zum Stechen einer menschlichen oder tierischen Haut, mit:
- einem Modulgehäuse,
- einem Koppelabschnitt, der an dem Modulgehäuse gebildet ist, um an ein Gehäuse (1) einer Antriebseinrichtung (2) des Handgeräts zu koppeln,
- einer Stecheinrichtung, die in dem Modulgehäuse mit einer Nadel (4; 15, 17) gebildet ist und direkt oder über eine Kopplungseinrichtung indirekt an die Antriebseinrichtung (2) des Handgeräts koppelbar ist, derart, dass zum Bewegen der Nadel (4; 15, 17) axial in Bezug auf das Modulgehäuse eine von der Antriebseinrichtung bereitgestellte Vorschubbewegung auf die Stecheinrichtung einleitbar ist,
- einer Modulgehäuseöffnung (6; 16), die an einer Nadeldüse (5) gebildet ist und zu der sich die Nadel (4; 15, 17) im Betrieb relativ vor und zurück bewegt, und
- einem Stechtiefeneinsteller (9; 18), bei dem zumindest ein distales Ende die Nadel (4; 15, 17) wenigstens abschnittsweise umgreift, der an die Stecheinrichtung koppelt und beim Vor- / Zurückbewegen der Nadel (4; 15, 17) im Betrieb mit dieser mitbewegt wird,
**dadurch gekennzeichnet, dass**
eine distale Endfläche des Stechtiefeneinstellers (9; 18) in einer Betriebsstellung gemeinsam mit einer vorderseitigen Endfläche der Nadeldüse (5) die Anschlagfläche für die Haut bildet und
eine freie Nadellänge, nämlich die Relativlage des Stechtiefeneinstellers (9; 18) in Bezug zur Nadelspitze und somit der Überstand der Nadel (4; 15, 17) über die distale Endfläche des Stechtiefeneinstellers (9; 18), einstellbar ist, indem der Stechtiefeneinsteller (9; 18) koaxial zur Nadel (4; 15, 17) und relativ zur Nadelspitze verlagerbar angeordnet ist.

2. Nadelmodul nach Anspruch 1, **dadurch gekennzeichnet, dass** die Nadel (4; 15, 17) an einem Nadelträger aufgenommen ist, der direkt oder über die Kopplungseinrichtung indirekt an die Antriebseinrichtung (2) koppelbar ist.

3. Nadelmodul nach Anspruch 1 oder 2, **dadurch gekennzeichnet, dass** der Stechtiefeneinsteller (9; 18) an der Nadel (4; 15, 17) in axialer Richtung verlagerbar aufgenommen ist.

4. Nadelmodul nach Anspruch 3, **dadurch gekennzeichnet, dass** der Stechtiefeneinsteller (9; 18) an der Nadel (4; 15, 17) an einem Nadelstößel (7) in axialer Richtung verlagerbar aufgenommen ist.

5. Nadelmodul nach mindestens einem der vorangehenden Ansprüche, **dadurch gekennzeichnet, dass** der Stechtiefeneinsteller (9; 18) über einen Drehstellmechanismus an die Stecheinrichtung koppelt.

6. Nadelmodul nach mindestens einem der vorangehenden Ansprüche, **dadurch gekennzeichnet, dass** einem an dem Stechtiefeneinsteller (9) gebildeten Führungselement (11) eine an der Nadeldüse (5) gebildete Führung zugeordnet ist, in welcher das Führungselement (11) beim Vor- / Zurückbewegen der Nadel (4; 15, 17) im Betrieb geführt ist.

7. Nadelmodul nach mindestens einem der vorangehenden Ansprüche, **dadurch gekennzeichnet, dass** ein Nadeldüsengehäusebauteil der Nadeldüse (5) verlagerbar an dem Gehäuse (1) angeordnet werden Kann und funktionell an den Stechtiefeneinsteller (9) koppelt.

8. Nadelmodul nach Anspruch 7, **dadurch gekennzeichnet, dass** das Nadeldüsengehäusebauteil drehbar an dem Gehäuse (1) angeordnet werden kann.

9. Nadelmodul nach mindestens einem der vorangehenden Ansprüche, **dadurch gekennzeichnet, dass** das distale Ende des Stechtiefeneinstellers (9) einen sich verjüngenden Endabschnitt aufweist.

10. Nadelmodul nach mindestens einem der vorangehenden Ansprüche, **dadurch gekennzeichnet, dass** der Stechtiefeneinsteller (9; 18) in einer oder mehreren axialen Verlagerungsstellungen relativ zur Nadelspitze der Nadel (4; 15, 17) fixierbar ist.

11. Handgerät zum Stechen einer menschlichen oder tierischen Haut, mit
- einem Gehäuse (1),
- einer Antriebseinrichtung (2), die in dem Gehäuse angeordnet und geeignet ist, wiederholt eine Vorschubbewegung bereitzustellen, und
- einem Nadelmodul nach mindestens einem der vorangehenden Ansprüche.

## Claims

1. A needle module (3) for a hand-held device for piercing a human or animal skin, comprising:
- a module housing,
- a coupling portion, which is formed on the module housing in order to be coupled to a housing (1) of a drive device (2) of the hand-held device,
- a needle device, which is formed in the module housing with a needle (4; 15, 17) and can be coupled directly or, via a coupling device, indirectly to the drive device (2) of the hand-held device in such a way that a feed motion provided by the drive device can be introduced onto the piercing device for moving the needle (4; 15, 17) axially with respect to the module housing,
- a housing opening (6; 16) which is formed on a needle jet (5), and relative to which the needle (4; 15, 17) moves forwards and backwards during operation, and
- a piercing depth adjuster (9; 18), which is coupled to the piercing device and is entrained with the needle as said needle moves forwards/backwards during operation, wherein at least a distal end of the piercing depth adjuster surrounds the needle (4; 15, 17) at least in portions,
**characterized in that**
a distal end face of the piercing depth adjuster (9; 18), in an operating position, forms a common stop face for the skin with a front end face of the needle jet (5), and
a free needle length, namely the relative position of the piercing depth adjuster (9; 18) with reference to the needle tip and thus the protrusion of the needle (4; 15, 17), can be set by arranging the piercing depth adjuster (9; 18) coaxially with the needle (4; 15, 17) and displaceably relative to the needle tip.

2. The needle module according to claim 1, wherein the needle (4; 15, 17) is received on a needle carrier, which is coupled directly or, via the coupling device, indirectly to the drive device (2).

3. The needle module according to claim 1 or 2, wherein the piercing depth adjuster (9; 18) is received on the needle (4; 15, 17) so as to be displaceable in the axial direction.

4. The needle module according to claim 3, wherein the piercing depth adjuster (9;18) is received on the needle (4; 15, 17) on a needle ram (7) so as to be displaceable in the axial direction.

5. The needle module according at least one of the preceding claims, wherein the piercing depth adjuster (9;18) is coupled via a rotary adjustment mechanism to the piercing device.

6. The needle module according at least one of the preceding claims, wherein a guide element (11) formed on a piercing depth adjuster (9) is assigned a guide formed on the needle jet (5), the guide element (11) being guided in said guide as the needle (4; 15, 17) moves forwards/backwards during operation.

7. The needle module according at least one of the preceding claims, wherein a needle jet housing component of the needle jet (5) can be arranged displaceably on the housing (1) and is functionally coupled to the piercing depth adjuster (9).

8. The needle module according to claim 7, wherein the needle jet housing component can be arranged rotatably on the housing (1).

9. The needle module according at least one of the preceding claims, wherein the distal end of the piercing depth adjuster (9) comprises a tapering end portion.

10. The needle module according at least one of the preceding claims, wherein the piercing depth adjuster (9; 18) can be fixed in one or more axial displacement positions relative to the tip of the needle (4; 15, 17).

11. A hand-held device for piercing a human or animal skin, comprising:
- a housing (1),
- a drive device (2), which is arranged in the housing and is suitable for repeatedly providing a feed motion, and
- a needle module according to at least one of the preceding claims.

## Revendications

1. Module d'aiguille (3) pour un appareil portatif destiné à piquer la peau d'un humain ou d'un animal, comprenant :
- un boîtier de module,
- une section d'accouplement formée sur le boîtier de module, destinée à s'accoupler à un boîtier (1) d'un dispositif d'entraînement (2) de l'appareil portatif,
- un dispositif de piquage formé dans le boîtier de module avec une aiguille (4 ; 15, 17) et apte à être accouplé directement ou indirectement par le biais d'un dispositif d'accouplement au dispositif d'entraînement (2) de l'appareil portatif de manière à pouvoir transmettre au dispositif de piquage un mouvement d'avance fourni par le dispositif d'entraînement pour déplacer l'aiguille (4 ; 15, 17) axialement par rapport au boîtier de module,
- une ouverture de boîtier de module (6 ; 16) formée sur un puits d'aiguille (5) et par rapport à laquelle l'aiguille (4 ; 15, 17) se déplace vers l'avant et vers l'arrière pendant le fonctionnement, et
- un dispositif de réglage de la profondeur de piquage (9 ; 18) dont au moins une extrémité distale entoure l'aiguille (4 ; 15, 17) au moins par tronçons, lequel est accouplé au dispositif de piquage et entraîné par l'aiguille (4 ; 15, 17) lors du mouvement avant/arrière de celle-ci pendant le fonctionnement,
**caractérisé en ce que**
une surface terminale distale du dispositif de réglage de la profondeur de piquage (9 ; 18) forme la surface de butée pour la peau ensemble avec une surface terminale avant du puits d'aiguille (5) dans une position de fonctionnement, et
une longueur d'aiguille, notamment la position relative du dispositif de réglage de la profondeur de piquage (9 ; 18) par rapport à la pointe d'aiguille et donc le dépassement de l'aiguille (4 ; 15, 17) au-delà de la surface terminale distale du dispositif de réglage de la profondeur de piquage (9 ; 18) peut être réglé en plaçant le dispositif de réglage de la profondeur de piquage (9 ; 18) de façon coaxiale par rapport à l'aiguille (4 ; 15, 17) et de façon mobile par rapport à la pointe d'aiguille.

2. Module d'aiguille selon la revendication 1, **caractérisé en ce que** l'aiguille (4 ; 15, 17) est reçue sur un support d'aiguille apte à être accouplé directement ou indirectement par le biais du dispositif d'accouplement au dispositif d'entraînement (2).

3. Module d'aiguille selon la revendication 1 ou 2, **caractérisé en ce que** le dispositif de réglage de la profondeur de piquage (9 ; 18) est reçu sur l'aiguille (4 ; 15, 17) de façon à pouvoir être déplacé dans la direction axiale.

4. Module d'aiguille selon la revendication 3, **caractérisé en ce que** le dispositif de réglage de la profondeur de piquage (9 ; 18) est reçu sur l'aiguille (4 ; 15, 17) sur un poussoir d'aiguille (7) de façon à pouvoir être déplacé dans la direction axiale.

5. Module d'aiguille selon l'une au moins des revendications précédentes, **caractérisé en ce que** le dispositif de réglage de la profondeur de piquage (9 ; 18) est accouplé au dispositif de piquage par le biais d'un mécanisme de réglage rotatif.

6. Module d'aiguille selon au moins une des revendications précédentes, **caractérisé en ce qu'**un guidage formé sur le puits d'aiguille (5), dans lequel l'élément de guidage (11) est guidé lors du déplacement avant/arrière (4 ; 15, 17) pendant le fonctionnement, est attribué à un élément de guidage (11) formé sur le dispositif de réglage de la profondeur de piquage (9).

7. Module d'aiguille selon l'une au moins des revendications précédentes, **caractérisé en ce qu'**une pièce de boîtier de puits d'aiguille du puits d'aiguille (5) peut être disposée de façon déplaçable sur le boîtier (1) et s'accouple fonctionnellement au dispositif de réglage de la profondeur de piquage (9).

8. Module d'aiguille selon la revendication 7, **caractérisé en ce que** la pièce de boîtier de puits d'aiguille peut être montée de façon rotative sur le boîtier (1).

9. Module d'aiguille selon l'une au moins des revendications précédentes, **caractérisé en ce que** l'extrémité distale du dispositif de réglage de la profondeur de piquage (9) présente une section terminale effilée.

10. Module d'aiguille selon l'une au moins des revendications précédentes, **caractérisé en ce que** le dispositif de réglage de la profondeur de piquage (9 ; 18) peut être fixé dans un ou plusieurs positions de déplacement par rapport à la pointe d'aiguille de l'aiguille (4 ; 15, 17).

11. Appareil portatif destiné à piquer la peau d'un humain ou d'un animal, comprenant :
- un boîtier (1),
- un dispositif d'entraînement (2) disposé dans le boîtier et approprié pour fournir un mouvement d'avance de façon répétée, et
- un module d'aiguille selon l'une au moins des revendications précédentes.
